# EUROPEAN PATENT APPLICATION

(11) **EP 2 272 523 A1**
(43) Date of publication of application: **12.01.2011**
(21) Application number: 10183169.1
(22) Date of filing: 30.08.2005
(51) Int. Cl.: A61K 36/48, A61K 35/20, A61K 35/54, A61P 1/04, A61K 31/198

(54) **Compounds, compositions, formulations and process for preparation thereof and method of treatment and management of acidity and related disorders**

(30) Priority: 01.09.2004 IN MU09472004
(62) Divisional of application: 05815862.7
(71) Applicant: Savangikar, Chitra Vasant, Mumbai- 400 018, Maharashtra (IN); Savangikar, Vasant Anantrao, Mumbai- 400 018, Maharashtra (IN)
(72) Inventor: Savangikar, Chitra Vasant, Mumbai- 400 018, Maharashtra (IN); Savangikar, Vasant Anantrao, Mumbai- 400 018, Maharashtra (IN)
(74) Representative: Ablett, Graham Keith

(57) **Abstract**

There is provided an alkaline derivative of an agent selected from a group comprising one or more or a mixture of or a combination of a protein, a peptide and a non-toxic amino acid, wherein the said alkaline derivative has been subjected to one or more of a treatment to remove excess of unreacted alkali. There is further provided a pharmaceutical dosage composition comprising the aforesaid alkaline derivative and a process for preparation of the aforesaid alkaline derivative.

## Description

This invention relates to new antacid products and processes for producing products for treating acidity and related ailments in human as well as animals.

Acidity and related gastro-intestinal disorders include acid refluxes, duodenal ulcers, stomach ulcers and other clinical conditions arising from increase in acidity. These conditions are treated mainly by means of conventional route of acid neutralization, the products which rule OTC (Over The Counter) market; or by a new approach of prevention of acid secretion based on H.sub.2 receptor antagonists or proton pump inhibitors, which are pharmaceutically active molecules and are prescription medicines.

As per US FDA, to qualify for an OTC composition as an antacid, unit dosage should have minimum of an Acid Neutralization Capacity of 5 milliequivalents. Acid Neutralization Capacity has been defined in "Remington: The Science and Practice of Pharmacy", Vol II, p. 1220, publishers Lippincott Williams & Wilkins, Philadelphia, 20th Edition, 2002 as "the number of milliequivalents of hydrochloric acid required to maintain 1 mL of an antacid suspension at pH 3 for 2 hr *in vitro*. This definition when read In terms of "unit dosage" to qualify for an OTC composition as antacid as per US FDA means a dosage that will neutralize 5 milliequivalents or more of hydrochloric acid and maintain its pH to 3 or above in 2 hours *in vitro.*

Acid neutralization is achieved in the Acid Neutralizing Antacid compositions mainly by resorting to use of one or a combination of compounds of alkali metals or alkaline earth metals. Occasionally such composition are supplemented by use of buffers to make them more effective. These inorganic acid neutralizing salts, although effective in relieving acidity related conditions, have their own side effects, which are worth avoiding if possible, but are inevitable unless viable alternative is found.

Never in the past was it anticipated that an acid neutralizing antacid composition mainly based on proteins and their derivatives shall be a viable alternative.

To explore a total or partial alternative to inorganic antacids totally or partially, work was carried out in this invention on potential and manner in which protein and protein derivatives can be used as antacids. Proteins and their derivatives are useful basically as nutrients. They have very few side effects, if at all, when compared to several side effects of inorganic antacids.

Undesirable side effects arising from inorganic antacids have been described elaborately in literature. A systemic antacid, such as sodium bicarbonate can cause alkalosis and electrolyte imbalances. Most of the antacids are derived from aluminium, calcium and magnesium, which are not so readily soluble. However, they form relatively insoluble compounds in the digestive tract and they are also absorbed systemically leading to toxicity. This absorption may enhance significantly in case of patients having ulcerative conditions. Ingestion of large amount of calcium can result in a milk-alkali syndrome which involves hypercalcemia, alkalosis and renal failure. Magnesium containing antacids in the long run can cause hyper magnesemia and diarrhea. Aluminium containing antacids in prolonged use can cause phosphate depletion, osteoporosis and osteomalacia as well as neurotoxicity. All the toxicities of aluminium-, calcium- and magnesium-containing antacids are common and more pertinent in patients with renal failure.

This invention provides antacids alternative to conventional inorganic antacids. This invention for the first time provides antacid compositions based mainly on untreated or hydrolysed or alkaline derivatives of proteins, protein concentrates, protein isolates, amino acids as pure individual or mixtures of several amino acids. The said alkaline derivatives of proteins, their hydrolysates, amino acids or of sodium salts of amino acids are novel products.

Use of the protein based antacids of this invention can also be made in conjunction of other inorganic antacids to make it possible to reduce the quantity of inorganic antacids required to be used, or with other Active Pharmaceutical Ingredient based antacid drugs to improve their efficacy, or in compositions with other Active Pharmaceutical Ingredients to help control acidity generate by them.

This invention provides a process of preparation of antacid compositions containing proteins, protein concentrates, protein isolates, protein hydrolysates, amino acids, amino acid derivatives as well as alkaline derivatives of proteins, protein concentrates, protein isolates, protein hydrolysates, amino adds and amino acid derivatives.

This invention also provides a method of treatment of acidity related disorders using the composition of the present invention.

This invention originated from a surprising observation that usual symptoms of hyperacidity, which included post meal burning sensation in the stomach, moderate headache, moderate backache etc. were relieved reproducibly within a minute whenever a wet, fiber free and water solubles free nutrient concentrate preparation, Leaf Protein Concentrate (LPC), extracted from leaves of fenugreek (Trigonella foenum-graecum L), a common green leafy vegetable (*Methi* in vernacular local language Marathi) was eaten even in as small quantity as less than 1 gram on dry basis. This LPC can be replaced for the purpose of the sensation of relief by any other protein concentrate or isolate derived from plant or animal sources.

Proteins and compositions rich in proteins in native form show very little acid neutralizing capacity in the form as they occur in the nature. It is perhaps for this reason that they were never anticipated as acid neutralizing agents. Yet they are useful as antacid agents. It is true that normal diet does have proteins as normal part of the diet and must be serving antacid functions when hydrolysed in stomach. Yet the idea of using protein concentrates as antacid agents is not the same as use of food proteins as part of food for antacid effects.

In hyperacidity conditions, what is required is management of more than normal secretion of acids over and above capability of protein component of normal diet to deal with them. Proteins form a small percentage of normal diet components, and for intake of an extra amount of protein to serve the! extra acidity neutralization requirement, if aimed to be met through normal diet components, will require impractical increase in the amount of normal dietary components that will have to be eaten and that too accompanied by unacceptable extra intake of calories. Further limitation is that proteins eaten as part of natural dietary components need not be all available for digestion and hydrolysis in the time they reside in stomach.

It is, however feasible to achieve extra intake of proteins, if isolates and concentrates of proteins having excellent Biological Value (Percentage of nitrogen retained in the body of the percentage of nitrogen absorbed in the digestive tract) extracted from their natural sources could be supplemented. This could be a first step in managing mild hyperacidity conditions and where prolonged control and slower onset of acid neutralization is desired. In addition to antacid effect, this protein supplementation shall serve beneficial nutritional purpose too, rather than side effects which inorganic antacids exhibit.

In this invention, it is seen that although in native form the buffering power of proteins is weak from chemical point of view, they are actually delayed release buffers. They are polymers of bipolar molecules with amino as well as carboxyl groups. Their real buffering power is bound in the form of peptide bonds which start getting released by peptic hydrolysis as soon as the protein reaches the stomach. This slow release of antacid activity is a feature which makes proteins far more superior to inorganic alkaline compounds as antacids, particularly because high alkalinity of inorganic alkaline antacids after acid neutralization results in increase in stomach alkalinity which results into an "acid rebound" i.e. stimulation of stomach to secrete more acid due to alkaline pH in the stomach.

In one embodiment of this invention, the acid neutralizing agent can be a protein concentrate or protein isolate or a synthetic protein. Protein concentrate or isolate are compositions of proteins usually prepared from natural sources by a process which results in products richer in protein than the original raw material. Being extracted from their natural raw materials, they are free from non-protein components and fully exposed to enzymatic action *in vitro* as well as when ingested in the stomach and the released hydrolysate shall impart its buffering effect in control of pH.

When more severe hyperacidity conditions are required to be handled and rapid release of already available antacid activity is desired, it would be more worthwhile to include in that embodiment of antacid treatment compositions, the peptic digests of the said isolates and concentrates of good Biological Value proteins or well balanced mixtures of amino acids derived from high Biological Value proteins. The digests could be produced in several different ways including using papain or any other proteolytic enzyme or could be an alkaline or acid digest done and protein degradation in such mixtures could have been up to various levels of degradation through a mixture of large polypetides through oligo peptides, tri- and di-peptides to amino acid level. Individual amino acids produced synthetically or by fermentation route may also be used directly in this case. These embodiments give effective acid neutralization capacity between pH 6.5 to pH 3.

Enzymatic protein digests may also be subjected to "plastein reaction", a reversal of hydrolysis catalysed by protelolytic enzymes under high unusually high substrate (a hydrolysate generated by proteolytic enzyme) concentration, of 50% or more, which generates products called "plasteins", which are also included as antacid active included in this invention. Plastein reaction is resorted to for achieving various objectives such as removal of bitterness and undesirable flavors (Fujimaki, M., Kato, H., Arai, S., and Tamaki, E. 1968. Food Technology, 22, page no. 889), for generation of agreeable flavors (Savangikar V.A. and Joshi, R.N. 1979. J.Sci.food Agric., 30, page no. 899), for generating therapeutic nutritional protein supplements (Yamashita, M., Arai, S., Tsai, S. -J. and Fujimaki, M. 1971. J. Agr. Food Chem. , 19, page no. 1151)

To handle still more severe conditions of hyperacidity, it was found in this invention that acid neutralization capacity of the untreated proteins and their hydrolysed derivatives including amino acids and sodium salts of amino acids and equivalent compounds can be improved markedly by making their alkali derivative, which are very useful for preparing antacid compositions. These alkali derivatives are novel products too.

The alkali treatment is possible in cold as well as hot condition and the alkali derivative formed having an alkaline pH are novel products. Sodium hydroxide treatment is most convenient for this purpose, although other alkalies may also be used and are covered in this invention. This results in an alkaline product which can be made frees from un-reacted alkali by titrating extra alkali above pH 10.8 by acid or by dialysis and such a dialysed product having alkaline pH (between pH 8 to 10.8) has further enhanced Acid Neutralization Capacity which sets rapidly as soon as ingested. Such an alkali derivative when it comes in contact with acids in stomach, shall return to its pre-treatment form, which is nothing but a normal nutrient in the form of native protein or peptides or amino acids having a beneficial effect and practically no or fewer side effects if at all, when compared to inorganic antacids. Far less quantity of such an alkali derivative will be needed to neutralize same quantity of acids in stomach and to comply with minimum requirements to qualify as antacid composition, such as the US FDA recommendations of minimum Acid Neutralization Capacity of 5 per dose.

It may be mentioned here that within the scope of this invention, amongst the options available, antacid effect of this invention would be available even from use of a single amino acid or its derivatives such as glutamic acid and sodium glutamate, which is included within the scope of this invention. However, this avenue is inferior to use of dietary proteins for this effect since its beneficial effect as nutrient besides antacid effect would be available only if the amino add is in a balanced amount with respect to other essential amino acids in the same diet, so that the absorbed amino acids are assimilated for tissue build up and repair and not excreted unassimilated or unutilized. This aspect brings in the focus preference: that should be given to high Biological Value dietary- proteins and their derivatives and balanced mixtures of essential (those amino acids which are not synthesized in animal body and must be supplied through food for normal protein metabolism) amino acids, which are characterized by high retention / assimilation inside the body after they are absorbed in the digestive tract, when use for the purpose of antacid application is the objective.

In normal circumstances, the additional protein that shall be ingested in antacid compositions is not significant enough to bother for balancing against other dietary protein sources. However, when protein intake is a limitation for a patient, such as in renal failures, it is possible to balance this extra input through isolates and concentrates of proteins by withdrawing appropriate quantity of protein contributing components of conventional diet including but not limited to pulses, meat, groundnut etc.

As a matter of course, basically this invention is valuable in usual hyperacidity patients to relieve them of side effects of inorganic antacids. However, over and above this, it is also valuable to most of the patients who are undergoing treatment on various other diseases who are temporarily vulnerable to acidity caused by intake of medicines prescribed to them. Inclusion of antacids based on proteins, their derivatives and alkaline derivatives shall be a very valuable way of relieving them from the side effects of acidity generated by medicine treatment to them on other disease conditions. Incidentally, most of the medicines that need to be ingested are known to cause hyperacidity. Thus, in most of the disease treatments, use of antacids of this invention either with the therapy or Incorporated in the unit dosage of the medicine may be valuable: as a normal course of treatment.

In practical course, it may be necessary to use two or more forms of the antacid actives covered in this invention to cover broad range of antacid protection: desired such as immediate onset as well as up to few hours after the meals.

In above assortment of protein and related products available, It is possible to visualize antacid preparations comprising proteins or their derivatives with or without alkaline treatment with total absence of alkali metal or alkaline earth metal compounds in such compositions, or with various proportions of the same in small quantity and also with or without buffers such as Potassium Hydrogen Phosphate.

The active ingredient, the isolates and concentrates of proteins, their hydrolysed derivatives and alkali derivatives of proteins and their hydrolysed products and amino acids, may be delivered to the patient through various solid as well as liquid dosage forms normally known in the art of pharmaceuticals including but not limited to powder dosage forms effervescent or otherwise, compacted forms such as granules and tablets of various forms and shapes, layered tablets, fast and slow release tablets, incorporated in capsules of slow release type or otherwise, syrups etc. The antacid active ingredient of this invention may also be incorporated in therapeutic food formulations for general healthcare, in a beverage or specific treatment including deriving an antacid effect, or with other pharmaceutically active ingredients in various dosage forms known in the art. This invention covers all pharmaceutical compositions described above and their analogous products obvious to a person in the field of art of pharmaceuticals and antacids in which proteins, their hydrolysates including individual amino acids and alkali derivatives of proteins and their hydrolysates and individual amino acids and the said compositions may contain one or more of other pharmaceutically acceptable Excipients, colors, flavors, binders, conventional as well as high intensity low calorie sweeteners including but not limited to saccharine, aspartem, acesulphame K, trichlorogalactosucrose (sucralose), stevioside, maltitol, lactitol, fructose, high fructose corn syrup, honey.

The said antacid ingredients of this invention including proteins, their hydrolysed and other derivatives including mixtures of or individual amino acids and alkali derivatives of proteins and their hydrolysed products including mixtures of amino acids or individual amino acids may be of natural origin in native or extracted form plants or animals or produced by bacterial fermentation or synthetically produced.

The description and examples, conditions of treatment provided here are for the purpose of illustrating the invention and any modification and improvements obvious to persons skilled in the art are also included in this disclosure of the claimed invention.

All dietary protein concentrates, isolates, their hydrolysates and their derivatives are as embodiments of this invention.

Of the several embodiments possible within the scope of the disclosure made here, some representative embodiments are mentioned below. The scope of the patent is not necessarily limited by the embodiments mentioned and any other embodiments within the scope of this invention are included herein.

Examples given below are illustrative for the purpose of explaining how the invention is to be performed. All protein concentrates, protein isolates, protein hydrolysates prepared by enzymatic or non-enzymatic methods, protein derivatives, protein hydrolysate derivatives, amino acids, amino acid mixtures, amino acid derivatives, amino acid mixture derivatives, Leaf Protein, Leaf Protein Concentrate, Leaf Nutrient Concentrate, Fraction I protein, Fraction II protein, Chloroplastic Protein, Cytoplasmic Protein, Fish Protein Concentrate, Fish Protein Isolate, Soya Protein Concentrate, Soya Protein Isolate, Groundnut protein concentrate, Groundnut Protein Isolate, Casein, Casein hydrolysate, Gelatin, Gelatin hydrolysate, egg protein, egg protein concentrate, egg protein isolate, glutamic acid, Single cell proteins from Spirulina etc, derived from plants, animal and microbial sources are included in this invention as embodiments of antacid actives useful in antacid compositions.

The examples given below illustrate the invention further. Active ingredients use, range of reaction conditions and range of reactants and other techniques used in the experiments are only illustrative and in no way limit the scope of the invention. Any variation obvious to a person skilled In the art and necessary for the purpose of scale up to commercial scale and optimal operation is included in the invention claimed here.

The proteins and related compounds described as antacid actives in above specification for the treatment of acidity and related disorders may be given in the form of a nutritional supplement or as a medication or both or given as a part of beverages too.

### EXAMPLE: 1

### MATERIALS & METHODS

### 1) PROTEIN RICH PREPARATIONS USEFUL IN ANTACID PREPARATIONS

Casein Hammerstein (isolate of milk protein) available from BDH Chemicals was used as one example of protein isolate from milk for illustrating this invention.

Protein concentrate extracted from juice of plant shoots (Leaf Protein Concentrate, also known as LPC) in native extracted form as water insoluble coagulum and in the form of its peptic hydrolysate is used to give an example from plant source as well as of use of a hydrolysed protein.

Several methods of preparation of LPC exist, which are covered and reviewed comprehensively by Pirie, 1971 (Pirie, N.W., 1971, IBP Handbook no. 20, Blackwell, Oxford). In the method used here, the protein concentrate is a heat precipitated (80 degrees celcius) coagulum of the juice expressed from pulp of the shoots of *Methi* (Trigonella foenum-graecum L.), a commonly eaten leafy vegetable in India. The pulp was made in a domestic kitchen mixer. The coagulum was washed several times in water, pressed free from excess water through cotton cloth, mixed with about 30% by weight of common salt and stored in refrigerator until used. Composition of such LPC, on dry weight basis, ranges between crude protein (Nitrogen muitiplied by 6) 45 to 55%. Crude fat (Ether Extract) 10 to 15%, crude fiber less than 1%, Total ash around 1 to around 6% and acid insoluble ash of about 1%.

The wet salt preserved LPC, immediately before use, was suspended in excess of water, allowed to settle and supernatant water decanted and discarded. The process was repeated until the water wash was free from salty taste. Finally, it was filtered through double layer cotton cloth, washed twice with water, hand squeezed free of excess of water and the wet coagulum was taken for use.

Dry matter: content of wet cake differed from batch to batch from between 20 to 37%.

The said LPC can be produced from any other leafy plant species and in a variety of different methods of production and any product analogous to' LPC produced by any process is covered in the embodiment of this invention for the purpose of application in antacid preparations. LPC is a very high Biological Value dietary protein. It has been subjected to several human feeding trials including children feeding trials and has been described as safe for human consumption. Pirie, 1971 referred above has covered a comprehensive information on LPC. Load of nitrogen and degradation products of protein metabolism that has to be disposed off through kidneys is, incidentally far less when high Biological Value protein is ingested than when a protein of low Biological Value is ingested.

### 2) REPARATION OF PROTEIN HYDROLYSATE - PEPTIC (ENZYMATIC)

Peptic digest of LPC was prepared in 0.1 N HCl by using pepsin (1:10,000) available from Difco. The digest was adjusted to pH 5.6, filtered through cotton cloth to get rid of undigested LPC, the filtrate concentrated and diluted appropriately by distilled water when required to be used.

Several methods are known in the art for enzymatic proteolysis. All these variations are included in the embodiment of this invention and may be followed for deriving hydrolysate for this invention.

### 3) PREPARATION OF ALKALI TRETAED ANTACID PRODUCT FROM ACTIVE INGREDIENTS SUCH AS PROTEIN, PROTEIN HYDROLYSATE OR AMINO ACIDS

About 20 ml of 1N NaOH was added to about or less than 1 gram dry weight of the active ingredient (Protein isolate or protein rich extract) with about 10 ml water content. In cold treatment, the mixture was kept at room temperature overnight until the sedimented coagulum was seen to have been dispersed evenly in a solution. In a hot treatment, the mixture was kept in an oven at 80 degrees celcius, volume restored to 30 ml by addition of distilled water and titrated after various stages of degradation after the period of treatment. Alkali remaining in excess was either neutralized by titrating against 1N HCl until pH dropped to 10.8 or by diaiyzing the mixture using gelatin membrane against 700 milliliter portions of distilled water three to four times until the dialysate contained little alkalinity.

The alkali treatment may, optionally, be terminated immediately after the addition of alkali, or continued for desired number of hours or days depending up on the degree of alkalinity of end product desired.

### DETERMINATION OF ACID NEUTRALIZATION CAPACITY (ANC) OF PROTEINS AND THEIR DERIVATIVES:

Acid Neutralization Capacity was noted as milliequivalents :of HCl neutralized to maintain the pH at 3 for two hours and expressed as per gram dry weight of the active antacid ingredient or per dose of the antacid composition containing the active ingredient. Total volume of the reactants were adjusted to 30 ml or 40 ml and titrated against 1N HCl and pH was noted after each small dropwise addition. The pH was measured by a pH meter.

In titration :of distilled water, it was observed that pH of the water raised rapidly to 10.8 with addition of a single drop. Similarly, while titrating 1N NaOH against 1N HCl, fall in pH from 12.2 to 10.8 was gradual. However, thereafter, just one drop was sufficient to lower the pH to neutral. Thus, it was clear that acid neutralized by the products within the pH range of 3 to 10.8 was due to Acid Neutralization Capacity when solutions of antacid active ingredients covered in the examples were titrated for determination of their ANC.

When alkali treated mixtures were titrated, acid required to bring down the pH to 10.8 was considered as acid required to neutralize the unreacted NaOH. Acid required to neutralize pH from 10.8 up to pH 3 was taken for calculation of ANC.

### DIALYSIS

Whenever desired, the alkali treated mixture was dialysed through gelatin membrane against about 700 milliliters of distilled water each time three to four times. Significant quantity of alkalinity appeared in first two dialyses in first two hours. Third dialysis, usually done for several hours overnight usually contained very little of alkalinity in the range of pH 10.8 and above.

### EXAMPLE 2

### ACID NEUTRALIZATION CAPACITY OF ACTIVE INGREDIENT

Following are the results of ANC of various antacid actives prepared and tested by methods given above:
Protein isolate of untreated Casein had ANC of 0.12 per gram.

The LPC showed ANC of 0.44 per gram dry matter. ANC of peptic digest of LPC was found to be 1.53 per gram dry matter. Thus, the actual ANC available from LPC when ingested even in its native form is not 0.24 gram per dry matter, but 1.53 gram per gram dry matter.

ANC of 1.5 per gram dry matter is practically relevant because a dose of about 4 gram at one time is practically feasible to be administered orally through tablets, effervescent powder formulations or flavored syrups, to enable delivery of a minimum ANC of 5 gram per dose.

Cold alkali treatment raised ANC of casein to 1.5. Hot alkali treatment of 2 days raised ANC to 4.88 and of 6 days to 8.

Cold alkali treatment raised ANC of LPC to 2.75. Hot alkali treatment of 4 hours raised ANC to 3.6, of one day to 5.5 and of 6 days to 7.9.

The un-reacted alkali In the alkali treated proteins can be either eliminated by titration against acid to lower the pH to 10.8. Dialysis of the alkali treated mixture through gelatin membrane against distilled water resulted in salt free and NaOH free alkaline product which ranged from pH of 10.6 to 8 depending on how much of low molecular weight component was lost in the water against which dialysis was made (wash water). When acid neutralized by the wash water below pH 10.8 and dry matter content of such washes (as corrected for weight of NaCl formed by NaOH neutralized above pH 10.8) was combined with the acid neutralized by the retaintate (solution retained inside the dialysis membrane) and dry matter content of the retaintate, it was seen that the combined ANC was around 6 for LPC in 4 hour treatment of hot alkali.

It is clear that alkali derivatives, cold as well as hot treated, of the proteins, their derivatives and amino acids are feasible antacid agents to give practical compositions with other ingredients to provide acid neutralization capacity of 5 or above. The untreated LPC shall still be useful as delayed release active because peptic digest of the same showed ANC of 1.5 between pH 5.6 to 3 and this capacity shall get reflected when digested in stomach. Same can be said to happen for casein too. The actual quantity of the actives of this invention used in an antacid composition will depend upon the local rules governing ANC of antacid preparations and requirements of the treatment of the disease indication aimed for alleviation.

### EXAMPLE3

### AMINO ACIDS AND THEIR ALKALINE DERIVATIVE:

About one milliequivalent of NaOH was neutralized per milliequivalent of Glutamic acid in water to raise the pH of its aqueous solution from 2.9 to about 5. Thereafter, there was rapid increase in pH to 8.4 followed by slower increase in pH and from.pH 5 to 10.9, about 1.2 milliequivalents of NaOH was consumed per milliequivalent of glutamic acid used. The product upto pH 5 is analogous to sodium glutamate formation, which in itself is analogous to an intermediate form of analogous protein derivative.

A product formed from the amino acid as well as protein above the range ' of pH 5 was novel and provided for substantially extra ANC.

### EXAMPLE 4

### SODIUM GLUTAMATE AND ITS ALKALINE DERIVATIVE

Crystalline Sodium Glutamate gave solution of pH 6.7. It took, per millliequlvalent of Sodium Glutamate used, little less than 1 milliequivalent of HCl to bring the pH from 6.7 down to 3 and about 1 milliequivalent of HCl to raise the pH from 6.7 to 10.8. Here, the crystalline Sodium Glutamate solution of pH 6.7 represents an intermediate form of alkaline protein. The form of Sodium Glutamate formed above pH 6.7 was novel and analogous to the alkaline form of protein in that pH range and both are novel products providing for substantially improved ANC.

Other aspects of the present invention include the following numbered aspects:
1. A composition for consumption of mammals including human being comprising:
   a. a pharmaceutically antacid effective quantity per dose of one or more of
      i. protein, natural, microbial or synthetic; including concentrates or isolates or processed food formis partly or fully isolated from their natural sources; and excluding when present as an integral part of milk, eggs, pulses, soybean, groundnut, meat, fish, cereals and other food items that are in their unprocessed or unmodified or normally cooked natural forms, and/or
      ii. protein degradation derivatives including hydrolysates obtained by treatment with acid, alkali, proteolytic enzymes and protein degradation products obtained by any other means and mixtures of or pure amino acids, and/or
      iii. protein derivatives including cold alkali treatment as well as hot alkali treatment, and/or
      iv. proteins in modified forms of their natural sources,and/or
      v. enzymatic derivatives from hydrolyzates by enzymatic molecular rearrangement products such as plasteins;
   b. optionally comprising one or more of pharmaceutically permitted additives, colors, antioxidants, vitamins, minerals, preservatives, buffers, Excipients, flavors, sweeteners;
   c. optionally comprising other Active Pharmaceutical Ingredients and other antacid agents.
2. An antacid composition of aspect no. 1 in which the said other Active Pharmaceutical ingredients and other antacid agents include one or more of antibiotics, analgesic, anti-pyretics, cardio-vascular drugs, anti-inflammatory drugs, muscle relaxants, H.sub.2 receptor antagonists, Proton pump inhibitors, drugs that enhance stomach mucosal protection, prokinetic drugs that enhance gastrointestinal motility, digestants, pancreatic enzymes and laxatives.
3. An antacid composition of aspect 1 wherein the said protein includes native form or isolated or extracted form of one or more of protein from plant or animal source including:
   a. casein
   b. leaf protein, leaf protein concentrate, leaf protein isolate, leaf nutrient concentrate extracted from any plant species,
   c. seed protein, seed protein concentrate, seed protein isolate including soy protein, soy protein concentrate, soy protein isolate, groundnut protein, groundnut protein concentrate, groundnut protein isolate,
   d. animal protein, animal protein concentrate, animal protein isolate including fish protein, fish protein concentrate, fish protein isolate, meat protein, meat protein concentrate, meat protein isotate, egg protein, egg protein concentrate, egg protein isolate, gelatin, gelatin hydrolysate,
   e. Microbial protein, microbial protein concentrate, microbial protein isolate including yeast, single cell protein, algal protein, spirulina, spirulina protein
   f. Amino acids produced by synthetic or fermentation process, and their derivatives, including Glutamic acid and Sodium Glutamate.
4. An antacid composition of aspect1 administered in one or more of solid, liquid or gel dosage forms including tablets, granules, powders, effervescent as well as non-effervescent forms, rapid release or slow release forms, encapsulated forms, syrups.
5. A composition of aspect 1 wherein the said protein hydrolysate is derived by enzymatic or non-enzymatic methods comprising one or more of alkaline hydrolysis, acid hydrolysis, hydrolysis by proteolytic enzymes including pepsin, papain, pancreatin, trypsin, chymotrypsin, bacterial proteases or degradation by mechanical means.
6. A composition of aspect 1 wherein the said protein derivative is one or more of an alkali treated derivative, enzymatically derived plastein product.
7. A process of preparation of the composition of aspect 1 wherein the said alkaline derivative is prepared by:
   a. adding alkali, including sodium hydroxide, to the protein and their derivatives of all types including peptides, amino acids or sodium derivatives of proteins, peptides, amino acids, to raise the pH to above 7, preferably above 12.2,
   b. optionally continuing alkali treatment until the protein solubilizes
   c. optionally continue the alkali treatment described in b. for several hours or several days,
   d. subjecting the preparation of step (b.) and (c.) to one or more of treatment to remove excess of unreacted alkali including molecular sieves, dialysis, molecular filtration, acid neutralization by titrating to pH up to or below pH 10.8 8. The alkaline derivative prepared by f the process of aspect 7.
9. A process of preparation of composition of aspects 1 to 6 comprising:
   a. Taking a protein, protein isolate, protein concentrate, high protein ingredient, protein derivative, protein hydrolysis product, mixture or pure amino acid,
   b. Optionally adding one or more of other inorganic antacids including Sodium Bicarbonate, Aluminium Hydroxide, Bismuth Salicylate, Calcium Carbonate, Dihydroxyaluminium Sodium Carbonate, Magnesium Hydroxide, Magnesium Oxide, Magnesium Trisilicate, Sodium Carbonate and mixtures thereof; buffers permitted in food including sodium acetate, Dipotassium hydrogen phosphate, potassium dihydrogen phosphate; a H2-Receptor antagonist in pharmaceutically effective quantity,
   c. Optionally adding one or more of pharmaceutically acceptable flavoring, excipients, coloring matters, additives, extenders, sweeteners, effervescence generating ingredients.
10. A method of treating acidity related disorders in mammals including human being by administering a composition of aspect 1.

## Claims

1. An alkaline derivative of an agent selected from a group comprising one or more or a mixture of or a combination of a protein, a peptide and a non-toxic amino acid, wherein the said alkaline derivative has been subjected to one or more of a treatment to remove excess of unreacted alkali.

2. An alkaline derivative of claim 1 having a pH of at least about 10 to about 10.8.

3. An alkaline derivative of claim 1 or 2 wherein the said treatment to remove excess of unreacted alkali comprises a molecular sieve, dialysis, molecular filtration or acid neutralization by titrating up to pH 10.8 to 10.

4. An alkaline derivative of any preceding claim comprising a sodium derivative or a potassium derivative.

5. An alkaline derivative of any preceding claim for oral consumption by mammals including a human, for use in treating gastric acidity related disorders.

6. A pharmaceutical dosage composition comprising an alkaline derivative of any preceding claim and one or more of:
a) one or more of a pharmaceutically permitted additive comprising a carrier, a color, an antioxidant, a vitamin, a mineral, a preservative, a buffer, an excipient, a flavor, a sweetener, a binder for tablet making; and
b) optionally containing one or more of another active pharmaceutical ingredient.

7. A composition according to claim 6 wherein said optional other active pharmaceutical ingredients include one or more of antibiotics, analgesics, anti-pyretics, cardio-vascular drugs, anti-inflammatory drugs, muscle relaxants, inorganic antacids, H₂ receptor antagonists, proton pump inhibitors, drugs that enhance stomach mucosal protection, prokinetic drugs that enhance gastrointestinal motility, digestants, pancreatic enzymes and laxatives.

8. A composition of claim 6 or 7 wherein the pharmaceutical dosage composition is in one or more of solid, liquid or gel dosage forms including tablets, granules, powders, effervescent as well as non-effervescent forms, rapid release or slow release forms, encapsulated forms, syrups.

9. A composition of any one of claims 6 to 8, wherein the pharmaceutical dosage composition is obtainable by a process comprising:
a) taking the said alkaline derivative;
b) optionally adding thereto one or more of other inorganic antacids including Sodium Bicarbonate, Aluminium Hydroxide, Bismuth Salicylate, Calcium Carbonate, Dihydroxyaluminium Sodium Carbonate, Magnesium Hydroxide, Magnesium Oxide, Magnesium Trisilicate, Sodium Carbonate and mixtures thereof; buffers permitted in food including sodium acetate, Dipotassium hydrogen phosphate, potassium dihydrogen phosphate; a H₂-Receptor antagonist in pharmaceutically effective quantity;
c) optionally adding thereto one or more of pharmaceutically acceptable flavoring, excipients, coloring matters, additives, extenders, sweeteners, effervescence generating ingredients.

10. A process of preparation of an alkaline derivative of any one of claims 1 to 5 comprising one or more of a following steps:
a) adding an alkali to one or more of an agent selected from a group comprising one or more or a mixture of a protein, a peptide and an amino acid to raise the pH to 10 or above, preferably above 12.2,
b) optionally continuing alkali treatment until the said agent solubilizes and preferably heating the reaction mixture to a preferred temperature of about 80°C,
c) optionally continuing the alkali treatment described in step b. of this claim for several hours or several days,
d) subjecting the preparation of step (b.) and ©.) of this claim to one or more of a treatment to remove excess of unreacted alkali, the said treatment comprising a molecular sieve, dialysis, molecular filtration, acid neutralization by titrating up to pH 10.8 to 10.
